# EUROPEAN PATENT APPLICATION

(11) **EP 4 260 804 A1**
(43) Date of publication of application: **18.10.2023**
(21) Application number: 22382347.7
(22) Date of filing: 11.04.2022
(51) Int. Cl.: A61B 5/16, A61B 5/318, A61B 5/369, A61B 5/00, G06F 3/01, G16H 20/70, A61B 5/0245, A61B 5/08, A61B 5/11

(54) **SYSTEM FOR CREATING AND MODULATING A VIRTUAL REALITY ENVIRONMENT FOR AN INDIVIDUAL**

(71) Applicant: Università di Pisa, 56126 Pisa (IT); Centre Suisse d'Electronique et de microtechnique SA, 2002 Neuchâtel (CH); Institute I3B Universitat Politècnica de València Polytechnic City of Innovation, 46022 Valencia (ES); Università di Roma Tor Vergata, 00133 Rome (IT); Commissariat à l'Energie Atomique et aux Energies Alternatives, 75015 Paris (FR); Universita' Degli Studi Di Padova, 35122 Padova (IT); Carli, Vladimir, 19273 Sollentuna (SE); Hadlaczky, Gergö, 17273 Sundbyb (SE); Quatechnion S.L., 46022 Valencia (ES)
(72) Inventor: Carli, Vladimir, 19273 Sollentuna (SE); Hadlaczky, Gergö, 17273 Sundbyb (SE); Valenza, Gaetano, 56122 Pisa (IT); Crettaz, Mathilde, 1004 Lausanne (CH); Haenni, Etienne, 2013 Colombier (CH); Correvon, Marc, 2087 Cornaux (CH); Dudnik, Gabriela Silvia, 2068 Hauterive (CH); Alcañiz Raya, Mariano, 46117 Betera (ES); Gascó Ortiz, Victor, 46184 Colinas de San Antonio, Valencia (ES); Ortega Perez, Mario, 46021 Valencia (ES); Parra Vargas, Elena, 46001 Valencia (ES); Toschi, Nicola, 00044 Frascati (IT); VAN WASSENHOVE, Virginie, 91400 Orsay (FR); Claudio, Gentili, 35138 Padova (IT); Cardi, Valentina, 35122 Padova (IT); Guixeres Provinciale, Jaime, 46006 Valencia (ES)
(74) Representative: Clarke, Modet y Cía., S.L.

(57) **Abstract**

System for creating and modulating a virtual reality environment (21) for an individual (1), according to their state of mind, cognitive load, attention level, emotional state, mood, stress level, anxiety level, perceived wellbeing, comprising projecting means (3) of a virtual reality environment (21); detecting means (4) of the state of mind of a individual (1); control means (5) for processing metrics (45), configured to determine the state of mind of the individual (1), and to adapt the virtual reality environment (21); the detecting means (4) being attached to the body (12) of the individual (1), allowing every individual (1) to share experiences, making real the complex interplay between multisensory information, and emotions experienced by another individual (1).

## Description

### TECHNICAL FIELD OF THE INVENTION

System for creating and modulating a virtual reality environment for an individual, according to their state of mind, cognitive load, attention level, emotional state, mood, stress level, anxiety level, perceived wellbeing, according to claims 1 to 16, incorporating notable innovations and advantages over technical solutions used so far.

### BACKGROUND OF THE INVENTION

It is currently known that, while social media may transmit and reveal one's emotional state and mood, the use of text, images, and videos exclusively strongly affect the phenomenology of who we are as feeling and thinking beings. This *other-than-in-person* perspective characterising current social interplay may play a fundamental role in mental health. With a potential lack of multisensory sources of information, the current digital social communication at the present moment significantly affects our ability to think about ourselves in the future, being biased by past experiences, episodic memories, and emotions.

Nowadays, immersive virtual reality (VR) creates interactive computer-generated worlds, which fully substitute real-world sensory perceptions with digital contents, hence producing the realistic perception of being in new life-sized environments. Virtual reality (VR) can produce sensory experiences and social interplay that would be nearly impossible to recreate in real life. For example, the British Museum in London reproduced a 4,000-year-old house to educate and attract new visitors to contraptions they would otherwise have only encountered behind a glass in the museum. Virtual reality (VR) is already used in sport events, putting for example viewers on the starting line and in the pits at last year's Daytona 500, as well as on the pitch at the National Hockey League. Virtual reality (VR) has also proved its effectiveness in several clinical settings and notably in the treatment of psychological disorders, giving rise to what is known as "Virtual Reality Exposure Therapy". This therapy allows for the creation of a safe virtual world, where the patient can experience new realities without feeling threatened, as well as to approach traumatic or phobic situations gradually to mitigate stress and anxiety.

An example of the application of virtual reality (VR) is described in patent KR20190074563, which is related to an apparatus for assessing and training people with suspected dementia including the aged, which let the people be assessed and trained in a realistic and interesting manner. The content providing terminal is configured to be connected to a VR device and a motion recognition device worn by the assessment subject (E) with/without wires. Training content run by the content providing terminal is shown to the assessment subject (E) in virtual reality via the VR device. The control unit causes, through processing, an avatar, which is moved in accordance with motions of the assessment subject (E), to be shown in the space of virtual reality in the VR device. The assessment subject (E) can be assessed and trained by controlling virtual objects with the avatar for a given mission.

Another related patent to the present invention is the US2014356830, which describes a method and system are presented to address quantitative assessment of facial emotion nulling of a subject, where the method comprises the steps of: i) presenting at least one scene, comprising a single facial expressions and a background, to a subject on a display; ii) adjusting the facial expression on the scene; iii) receiving feedback from the subject via at least one input device; iv) quantitatively refining the received feedback; v) modulating the adjusted facial expression relative to the accuracy of the quantitatively refined feedback; vi) transforming the modulated facial expression; vii) calculating a critical threshold parameter; and viii) recording a critical threshold parameter onto a tangible computer readable medium.

However, there are no prior art disclosures aimed specifically to widespread the virtual reality (VR) for a new interpersonal communication and exploitation of the future digital world. Allowing every person to share experiences, making virtually real the complex interplay between multisensory information, and the emotions experienced by another person, are, as of yet, uncharted innovations. This approach will augment the future digital social interactions by manipulating one's internal representation of virtual space-time, radically changing the phenomenological interplay in its physiological and affective dimensions.

### DESCRIPTION OF THE INVENTION

The present invention consists of a hardware and software technology allowing everyone to generate their own virtual reality (VR) environment. Each single person becomes a provider. A new virtual reality (VR) environment will also be automatically generated from personal neurophysiological data of the individual provider and will be equipped with psychological, cognitive, neurophysiological, and behavioural information to constitute what it is called the person's Extended-Personal Reality. Said Extended-Personal Reality may be shared with other persons, the users, for a new kind of personal experience and social interaction. The user will relive the shared provider's experience through VR-based, visual-auditory and tactile stimuli, space-time manipulation, and multiple biofeedbacks. The invention application shall effectively communicate the provider's emotions and/or subjective feelings to allow access and to enhance previously unreachable consciousness layers.

The person's Extended-Personal Reality concept of the present invention is defined as the set of all neurophysiological, psychological, cognitive, and behavioural responses occurring in a given recording in space and time. This set includes the subject's mood/emotional as well as cognitive state with respect to their time perception and its disentanglement from spatial cognition by visual / audio / haptic stimulation reality.

The invention application concept and methodology will be founded on, first, a technological foundation, comprising new wearable devices for recording the user's EEG, ECG, respiration, environment vision and sound, movement and hand kinematics, as well as to mark/annotate significant events. Furthermore, this includes the invention application interpretable Artificial Intelligence (Al) engine and the novel user-friendly software for automatic creation of VR environments. Second, in scientific foundation, comprising fundamental tools for the creation and definition of an individual's Extended-Personal Reality, allowing for the estimation of the individual's emotional, as well as cognitive state with respect to their space-time perception. A new generation of multimodal neuroimaging / electrophysiological biomarkers from fMRI/MEG/EEG, as well as from autonomic nervous system dynamics are devised. Third, a scientific foundation comprising the set of multisensory elicitation strategies needed to allow a user to live an experience from a person's Extended-Personal Reality. Unprecedented emotional elicitation using techniques based on combined video/audio/haptic stimuli, Artificial Intelligence (Al) guided multiple biofeedbacks, and neuromodulation as linked to subjective time and representation of self are implemented. An Al-based, fully generative engine from neurophysiological data for VR-based recall paradigm is created as well. And fourth, the application in a clinical scenario, comprising methodological tools for the redefinition of psychiatric illnesses according to the patient's Extended-Personal Reality features. In particular, a new framework is provided for the classification and treatment of the clinical spectrum of psychiatric disorders, unveiling previously undiscovered subtypes of anxiety, depression, and eating disorders.

More specifically, the invention is related to a system for creating and modulating a virtual reality environment for an individual, according to their state of mind, cognitive load, attention level, emotional state, mood, stress level, anxiety level, perceived wellbeing. This invention comprises: projecting means of a virtual reality environment around the individual; detecting means of the state of mind of the individual, configured to establish at least one metric proportional to a neuro-physiological variable and/or a behavioural variable of the individual; control means for processing said metric, configured to determine the state of mind of the individual, and to adapt the virtual reality environment to said state of mind of the individual according to predetermined conditions based on said metric; the detecting means being configured to be attached to the body of the individual. In this way it is possible to adapt what is perceived by the individual, in order to improve either his state of mind or psychological state, or his mental health, or equivalent.

Just to precise, that state of mind of the individual has to be understood as any of arousal and vigilance, anxiety, mood, tiredness, cognitive stamina and performance levels. Said state of mind, or equivalents as cognitive load, attention level, emotional state, mood, stress level, anxiety level, perceived wellbeing, may be modelled through a combination of neural, physiological, cognitive, behavioural, and vision/haptic/audio information. In particular the system will acquire signals from both the autonomic nervous system (e.g., ECG, respiration), and the central nervous system (e.g., EEG), and will combine them with behavioural data including accelerometer/gyroscope outputs, eye tracking/gaze, and video recordings to perform an automatic recognition of state of mind, cognitive load, attention level, emotional state, mood, stress level, anxiety level, wellbeing.

In addition, the system for creating a virtual reality environment for an individual includes detecting means that comprise at least one head sensor placed in a helmet, such that the system detects variables of the environment similar to what is being perceived by the individual.

The helmet, in a particular embodiment, may comprise an inner cap and an outer cap. And said head sensor, being an electrode to be placed over the subject's scalp, is preferably located in the inner cap, for a better detection of a physiological variable of the individual's head. On the other hand, the outer cap, in another particular embodiment, can be a headband with one camera on the forehead, and in an alternative embodiment can support two cameras providing stereoscopic recordings. In this way the audio and video data logger of said two cameras will record two synchronised images and sound, being able of being recorded in high density SD memory cards.

Complementarily, the detecting means comprise at least one chest sensor placed in a body harness, in this way placed close to the heart and/or lungs, and therefore being able to detect a physiological variable of the individual related to his breathing and heart rate. Preferably, said chest sensor lodges associated electronics comprising, in a particular embodiment, Electrocardiography electrodes, for a better detection of the heart rate.

More in detail, the detecting means comprise data capturing means, so that the system for creating a virtual reality environment may regulate and selected what data of the individual's physical environment is going to be captured specifically, in every moment.

Advantageously, the data capturing means comprise an audio recorder and/or a video recorder of the physical environment of the individual, so that the sound and/or the images surrounding the individual can be captured and recorded for a better selection or recreation, for instance, of a virtual reality (VR) effect. In a particular embodiment, the audio recorder and/or a video recorder may be a stereoscopic camera, i.e.: two or more cameras, and microphones that provide a stereoscopic view and acquisition of the environmental sound.

Furthermore, the data capturing means comprise a movement sensor of the body of the individual, so that the system can determine the position of said individual in a particular environment, and his variations of position, and the speed of the variations, for a better diagnosis of the state of mind, interests or mood of the individual. Particularly the movement sensor may comprise any of head, hands or eyes movement sensor. All the date gathered by these movement sensors can be processed for a better determination of the state of mind, interests or mood of the individual.

In addition, the control means comprise a haptic switch placed in a body harness, enabling the individual to mark events during the monitoring session by tapping a specific number of times on the enclosure.

More particularly, the system for creating a virtual reality environment for an individual, comprises data recording means, so that the specific data for an individual along can be recorded over a period of time, giving a better perspective of the state of mind, interests or mood of said individual, being possible a better diagnosis upon comparing present data with date of the past. Said data recording means is optionally an embedded memory mass, preferably placed in a body harness, so that the individual can carry the data recording means along the way he is moving, without transmitting continuously said data to a remote recording means.

Optionally, the control means and/or the data recording means are removably attached to a body harness, so that said data recording means can, firstly, gather locally the data in the assembled position on the body harness, without the need of transmitting them, and secondly, to download afterwards the gathered data to a further device, in a disassembled position of the body harness, being optionally assembled to said further device.

Preferably, the system for creating a virtual reality environment for an individual comprises a docking station, as a further device, for the control means and/or the data recording means. Said docking station has the functions of data upload and/or download, and as recorder battery recharge. The data, particularly the physiological data, captured by the detecting means, and gathered by the data recording means are uploaded to the cloud via the docking station and an access point or router.

Complementarily, the docking station comprise communication means and/or electric supply means, so that, as mentioned, thanks to the communication means, the data can be uploaded, for example, to the cloud, via additionally an access point or router. Said communication means are particularly transmitting means. And thanks to the electric supply means, the control means and the data recording means, integrated optionally in an electronic module, may be electrically recharged, so that they can work on the body harness, and apart from the docking station. Particularly the electric supply means of the docking station may be an AC/DC adapter for an easy electric supply from the electrical grid.

Additionally, the system for creating a virtual reality environment for an individual comprises communication means with associated visualisation means, so that some variables or data can be viewed by the individual himself, as user of the system for creating a virtual reality environment, or by an external manager of the system.

Further, the communication means comprises signal transmitting means between a helmet and/or a body harness and/or associated visualisation means and/or an associated docking station, so that several elements of the system may be in communication, transmitting data between them.

Complementarily, the communication means comprises synchronisation means of the signals transmitted by the signal transmitting means between the helmet and/or the body harness and/or the associated visualisation means and/or the associated docking station, so that several elements of the system may be synchronised, working at a same speed, frequency, and in a coordinated way.

Additionally, the projecting means comprise virtual reality glasses, so that the individual, as user of the system for creating a virtual reality environment, enjoys of a more complete visual perception of a virtual reality.

Optionally, the projecting means comprise a haptic interface, so that the individual, as user of the system for creating a virtual reality environment, enjoys of an additional perception of a virtual reality by way of tactile feelings. Biofeedback may be provided also through haptic interfaces to increase the virtual reality (VR) realism and thus inmersiveness during the interaction with virtual or remote environments / machines; include fabric-based wearable systems to deliver normal force and contact-pressure cues at the finger pad level.

In the attached drawings it is shown, by way of non-limiting example, a system for creating a virtual reality environment for an individual, constituted according to the invention. Other characteristics and advantages of said system for creating a virtual reality environment for an individual, object of the present invention will become apparent from the description of a preferred, but not exclusive, embodiment that is illustrated by way of non-limiting example in the accompanying drawings, in which:

### BRIEF DESCRIPTION OF THE DRAWINGS

Figure 1.- Is a diagram with the elements comprised in the system for creating a virtual reality environment for an individual, in accordance with the present invention.
Figure 2.- Is a detail of the elements of a system for creating a virtual reality, weared by an individual, according to the present invention.
Figure 3.- Is a further detail of the elements of a system for creating a virtual reality, weared by an individual, according to the present invention.
Figure 4.- Is a detail of the representations of a system for creating a virtual reality, according to the present invention.

### DESCRIPTION OF A PREFERRED EMBODIMENT

In view of the aforementioned figures and, according to the numbering adopted, a preferred embodiment of the invention can be seen in them, which comprises the parts and elements indicated and described in detail below.

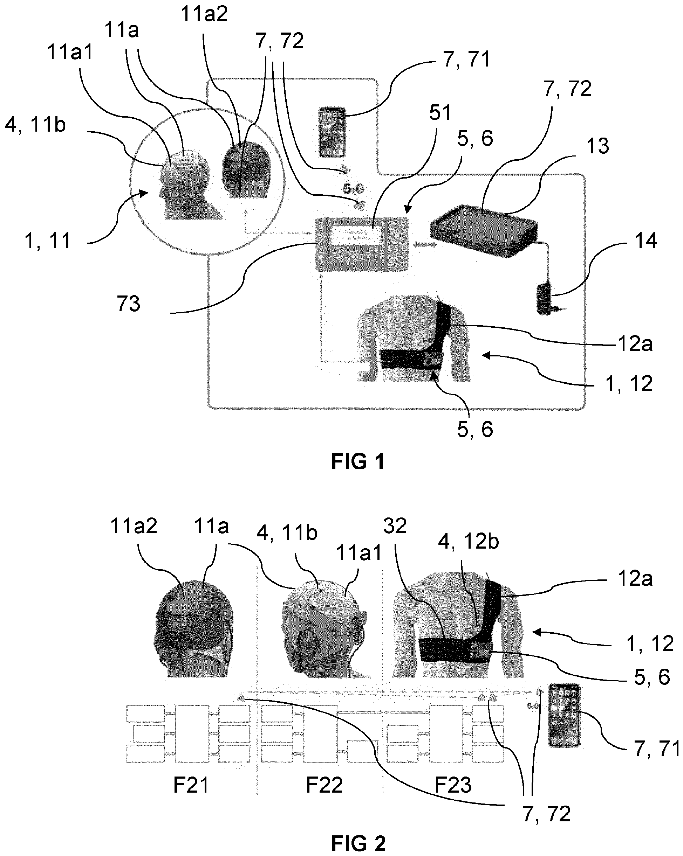

Figure 1 illustratively shows a diagram with the elements comprised in the system for creating a virtual reality environment for an individual. And Figure 2 illustratively shows a detail of the elements of a system for creating a virtual reality, weared by an individual. Said wearables, psychometrics, and biofeedback for recording date relative to an VR experience develops, in a preferred embodiment, medium risk/high reward advanced technology based on active electrodes improving signal quality with limited cabling, as intended for EEG, ECG, electrodermal activity, and Impedance PlethysmoGraphy-respiratory (IPG) monitoring. This new technology allows to record experiences in terms of audio/video/tactile information and associated psychophysiological patterns, and to benefit of the real-time, multiple biofeedback capability.

Also the present invention application proposes to integrate attitude monitoring (Quaternions or Euler angles) into each device. Stereoscopic cameras and microphones will provide a stereoscopic view and acquisition of the environmental sound, respectively, and two options are available: i) the recording capability on the dedicated wearable data logger (embedded memory mass), ii) the real-time raw data transfer to a gateway (e.g., tablet/smartphone) through, e.g., Bluetooth 5.0. Real-time Artefacts identification and rejection: Among the causes of biological artefacts are eye movements, blinks, muscle activity and heartbeat. Electrically induced artefacts can be caused by high electrode impedance, line noise, and susceptibility to electromagnetic interference.

Further the present invention application develops a new electrode concept with a low impedance channel to guide the disturbance current, improving the immunity to the earth's electric field (100 V/m) as well as to line noise and all other kind of interferences. Regarding signal synchronisation, Bluetooth 5.0 is used to synchronise the raw signals from the outer cap (11a2) (video and audio), the inner cap (11a1) (EEG) and the body harness (12a) or chest belt (ECG/IPG). Regarding signal quality, usability, and autonomy, a new mixed-signal ASIC is used with the aim of reducing size and power consumption. Moreover, an adaptive analog frontend filter enables a robust bio potential (EEG, ECG) baseline during intensive motion as well as the computing of a quality index assessing that the raw signals are in the nominal operating range.

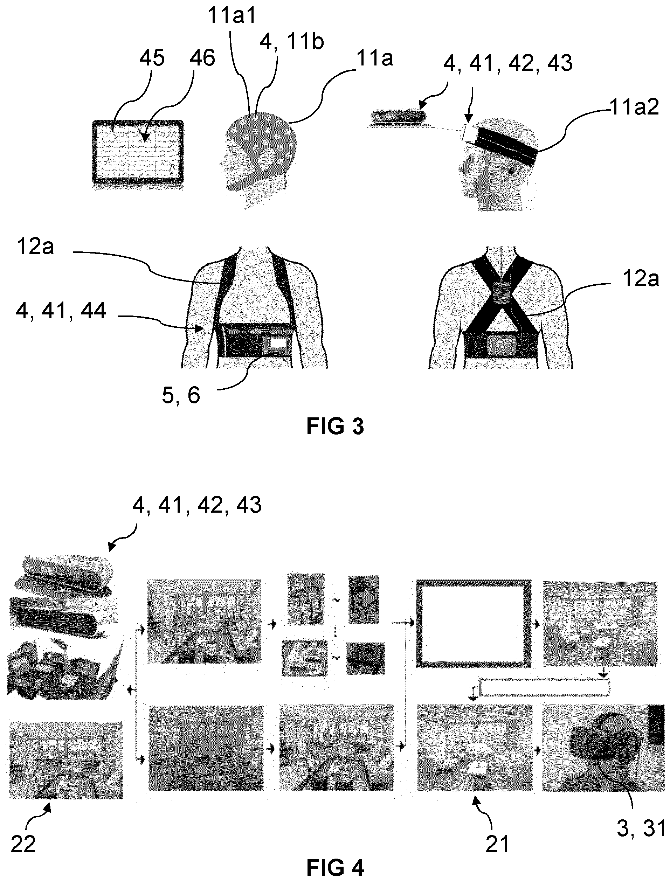

Figure 3 illustratively shows a further detail of the elements of a system for creating a virtual reality, weared by an individual. Regarding the multimodal neuroimaging/physiological framework of cognition as related to space, the simultaneous use of MEG and EEG allows low risk/high gain recording of the generators of brain activity that one or the other technique may be blind to. EEG is sensitive to both tangential and radial components of the generated electromagnetic fields, whereas MEG is most sensitive to its tangential component, and blind to radial ones. These non-invasive techniques record the magnetic and electrical activity of neural populations of the brain with exquisite temporal resolution on a millisecond scale. Source reconstruction (~1 cm) is estimated on the basis of individual anatomical MRI and informed by fMRI activity. fMRI allows the application invention to integrate the excellent time resolution provided by EEG and MEG with high spatial resolution (2mm) imaging that also includes activity of limbic/subcortical regions, otherwise not accessible in terms of oscillatory signals recorded from the scalp. Autonomic markers are derived to further characterise "stressors" (or other stimulus-induced neuro-physiological state) at a brain-heart level. As KPI, all experiments will systematically collect reaction times measurements and responses (e.g., by button presses), enabling to assess individual performances, error rates, perceptual thresholds and sensitivity.

Figure 4 illustratively shows a detail of the representations of a system for creating a virtual reality, for the public at large. The customisation of a 3D scenario in VR is extremely important. This task is usually performed manually from a previously created 3D scenario, or by creating a scenario following the specifications by the therapist. This requires a significant number of hours by an expert 3D graphical designer/programmer. Thanks to its advances in artificial intelligence (Al), the invention application enables any non-expert user to perform this 3D customisation. To achieve this automation, it is used: style transfer, semantic analysis of the scene, detection and pose estimation of 3D objects and 3D objects retrieval. Non-expert users only have to select the 3D objects that they wish to replace along with their replacements and select from which 3D scenario they may wish to copy a style. This will further enhance the personalisation of VR for clinical treatments, as well as improve the effectiveness of the emotional elicitation. More specifically, starting from the unique data gathered through the healthcare wearables, a combination of advanced computer vision techniques and deep learning algorithms is employed for the fully automatic creation of VR experiences. The methodology followed starts with the image and depth acquisition with wearable cameras. Then, a dense 3D scene can be reconstructed using camera pose estimation algorithms. The next step is to clean and simplify the scene extracting the semantic information. Then, this scene is analysed by detecting the objects and extracting the room layout. Using these data, a realistic scene is then rendered where previously modelled objects can be replaced. An important step is the style transfer algorithm, which blends the objects with the real scene and changes the overall appearance of the 3D scene. The highly personalised scenario (3D VR + space-time dimension) can be precise, photorealistic, customisable, and efficiently adapted to multiple VR systems. Finally, this tool allows non experts to create these high-end, high-quality scenarios in a simple way.

More particularly, according to figure 1, a system for creating and modulating a virtual reality environment (21) for an individual (1), according to their state of mind, cognitive load, attention level, emotional state, mood, stress level, anxiety level, perceived wellbeing, comprises projecting means (3) of a virtual reality environment (21) around the individual (1); detecting means (4) of the state of mind of the individual (1), configured to establish at least one metric (45) proportional to a neuro-physiological variable and/or a behavioural variable of the individual (1); control means (5) for processing said metric (45), configured to determine the state of mind of the individual (1), and to adapt the virtual reality environment (21) to said state of mind of the individual (1) according to predetermined conditions (46) based on said metric (45); and the detecting means (4) being configured to be attached to the body (12) of the individual (1).

Additionally, according to figure 3, the detecting means (4) comprise at least one head sensor (11b) placed in a helmet (11a), said head sensor (11b) configured particularly to EEG Electroencephalography, as an example, a 10/20 configuration with advanced active electrodes and a dedicated module as signal acquisition front-end at the basis of the occipital lobe.

Complementarily, according to figure 2, the detecting means (4) comprise at least one chest sensor (12b) placed in a body harness (12a).

Optionally, according to figure 3, the detecting means (4) comprise data capturing means (41).

More specifically, according to figure 4, the data capturing means (41) comprise an audio recorder (42) and/or a video recorder (43) of the physical environment (22) of the individual (1), such that a 3D scene can be reconstructed using camera pose estimation algorithms. The audio recorder (42) includes optionally a CCD (Charge-Coupled Device) microphone, and the video recorder (43) includes optionally an AHRS (Attitude and Heading Reference System).

In addition, according to figure 3, the data capturing means (41) comprise a movement sensor (44) of the body (12) of the individual (1).

Advantageously, according to figure 1, the control means (5) comprise a haptic switch (51) placed in a body harness (12a).

Furthermore, according to figure 3, the system for creating a virtual reality environment (21) for an individual (1) comprises data recording means (6), being optionally a data logger with any of EEG, ECG, Accelerometer, and an event marker.

More specifically, according to figure 3, the control means (5) and/or the data recording means (6) are removably attached to a body harness (12a).

In a preferred embodiment of the invention, according to figure 1, the system for creating a virtual reality environment (21) for an individual (1) comprises a docking station (13) for the control means (5) and/or the data recording means (6).

Preferably, according to figure 1, the docking station (13) comprises communication means (7) and/or electric supply means (14).

In a preferred embodiment of the invention, according to figure 1, the system for creating a virtual reality environment (21) for an individual (1) comprises communication means (7) with associated visualisation means (71). The communication means (7) may comprise a gateway in the docking station plus an access point or router. The visualisation means (71) may be a tablet or smartphone for data visualization and session synchronization. Said tablet or smartphone synchronizes the physiological data recorder or data capturing means (41) and the camera recorder or video recorder (43).

Complementarily, according to figure 2, the communication means (7) comprises signal transmitting means (72) between a helmet (11a) and/or a body harness (12a) and/or associated visualisation means (71) and/or an associated docking station (13).

Optionally, according to figure 1, the communication means (7) comprises synchronisation means (73) of the signals transmitted by the signal transmitting means (72) between the helmet (11a) and/or the body harness (12a) and/or the associated visualisation means (71) and/or the associated docking station (13). Preferably, the standard protocol Bluetooth 5.0 is used to synchronise the raw signals from the outer cap (11a2), of video and audio, the inner cap (11a1), of EEG, and the chest belt, with an ECG sensor, or accelerometer sensor or an event marker.

Alternatively, according to figure 4, the projecting means (3) comprise virtual reality glasses (31).

In another aspect of the invention, according to figure 2, the projecting means (3) comprise a haptic interface (32).

The details, shapes, dimensions and other accessory elements, as well as the components used in the system for creating a virtual reality environment for an individual, may be conveniently replaced by others that are technically equivalent., and do not depart from the essential nature of the invention or from the scope defined by the claims that are included in the following list.

### List of numerical references:

- 1: individual
- 11: head
- 11a: helmet
- 11a1: inner cap
- 11a2: outer cap
- 11b: head sensor
- 12: body
- 12a: body harness
- 12b: chest sensor
- 13: docking station
- 14: electric supply means
- 21: virtual reality environment
- 22: physical environment
- 3: projecting means
- 31: virtual reality glasses
- 32: haptic interface
- 4: detecting means
- 41: data capturing means
- 42: audio recorder
- 43: video recorder
- 44: movement sensor
- 45: metric
- 46: conditions
- 5: control means
- 51: haptic switch
- 6: data recording means
- 7: communication means
- 71: visualisation means
- 72: transmitting means
- 73: synchronisation means

### Text in figures:

| | |
|---|---|
| F21 | audio and video recorder (outer cap) |
| F22 | EEG electrodes + AFE (inner cap) |
| F23 | ECG-IPG-Event Marker (harness) |

### Being

| | |
|---|---|
| AFE: | Analog Front-End |
| EEG: | Electro-EncephaloGraphy |
| ECG: | Electro-CardioGraphy |
| IPG: | Impedance PlethysmoGraphy |

## Claims

1. System for creating and modulating a virtual reality environment (21) for an individual (1), according to their state of mind, cognitive load, attention level, emotional state, mood, stress level, anxiety level, perceived wellbeing, comprising:
- projecting means (3) of a virtual reality environment (21) around the individual (1);
- detecting means (4) of the state of mind of the individual (1), configured to establish at least one metric (45) proportional to a neuro-physiological variable and/or a behavioural variable of the individual (1);
- control means (5) for processing said metric (45), configured to determine the state of mind of the individual (1), and to adapt the virtual reality environment (21) to said state of mind of the individual (1) according to predetermined conditions (46) based on said metric (45);
- the detecting means (4) being configured to be attached to the body (12) of the individual (1).

2. System for creating a virtual reality environment (21) for an individual (1), according to claim 1, **characterized in that** the detecting means (4) comprise at least one head sensor (11b) placed in a helmet (11a).

3. System for creating a virtual reality environment (21) for an individual (1), according to any of the preceding claims, **characterized in that** the detecting means (4) comprise at least one chest sensor (12b) placed in a body harness (12a).

4. System for creating a virtual reality environment (21) for an individual (1) according to any of the preceding claims, **characterized in that** the detecting means (4) comprise data capturing means (41).

5. System for creating a virtual reality environment (21) for an individual (1), according to claim 4, **characterized in that** the data capturing means (41) comprise an audio recorder (42) and/or a video recorder (43) of the physical environment (22) of the individual (1).

6. System for creating a virtual reality environment (21) for an individual (1), according to any of the claims 4 or 5, **characterized in that** the data capturing means (41) comprise a movement sensor (44) of the body (12) of the individual (1).

7. System for creating a virtual reality environment (21) for an individual (1), according to any of the preceding claims, **characterized in that** the control means (5) comprise a haptic switch (51) placed in a body harness (12a).

8. System for creating a virtual reality environment (21) for an individual (1), according to any of the preceding claims, **characterized in that** it comprises data recording means (6).

9. System for creating a virtual reality environment (21) for an individual (1), according to claim 8, **characterized in that** the control means (5) and/or the data recording means (6) are removably attached to a body harness (12a).

10. System for creating a virtual reality environment (21) for an individual (1), according to claim 8, **characterized in that** it comprises a docking station (13) for the control means (5) and/or the data recording means (6).

11. System for creating a virtual reality environment (21) for an individual (1), according to any of the claims 10, **characterized in that** the docking station (13) comprise communication means (7) and/or electric supply means (14).

12. System for creating a virtual reality environment (21) for an individual (1), according to any of the preceding claims, **characterized in that** it comprises communication means (7) with associated visualisation means (71).

13. System for creating a virtual reality environment (21) for an individual (1), according to claim 12, **characterized in that** the communication means (7) comprises signal transmitting means (72) between a helmet (11a) and/or a body harness (12a) and/or associated visualisation means (71) and/or an associated docking station (13).

14. System for creating a virtual reality environment (21) for an individual (1), according to claim 13, **characterized in that** the communication means (7) comprises synchronisation means (73) of the signals transmitted by the signal transmitting means (72) between the helmet (11a) and/or the body harness (12a) and/or the associated visualisation means (71) and/or the associated docking station (13).

15. System for creating a virtual reality environment (21) for an individual (1), according to any of the preceding claims, **characterized in that** the projecting means (3) comprise virtual reality glasses (31).

16. System for creating a virtual reality environment (21) for an individual (1), according to any of the preceding claims, **characterized in that** the projecting means (3) comprise a haptic interface (32).
